# EUROPEAN PATENT APPLICATION

(11) **EP 4 563 538 A1**
(43) Date of publication of application: **04.06.2025**
(21) Application number: 23846982.9
(22) Date of filing: 26.07.2023
(51) Int. Cl.: C02F 1/32

(54) **FLUID TREATMENT DEVICE**

(30) Priority: 27.07.2022 US 202263392789 P
(71) Applicant: Seoul Viosys Co., Ltd., Ansan-si, Gyeonggi-do 15429 (KR)
(72) Inventor: JEONG, Jae Hak, Ansan-Si Gyeonggi-do 15429 (KR); CHOI, Eun Mi, Ansan-Si Gyeonggi-do 15429 (KR)
(74) Representative: Stolmár & Partner Patentanwälte PartG mbB
(86) International application number: PCT/KR2023/010792
(87) International publication number: WO 2024/025324

(57) **Abstract**

A fluid treatment device is disclosed. The fluid treatment device includes: a housing; an inner tube unit disposed inside the housing; and a light source unit emitting light to an inner space of the housing. The housing includes an inlet port through which a fluid is introduced and an outlet port through which the fluid is discharged. The inner tube unit has a smaller inner diameter than the housing and is open at a lower end thereof. An inner space of the inner tube unit defines a first flow path, and a space between the inner tube unit and the housing defines a second flow path. The fluid introduced through the inlet port flows to the outlet port after passing through the first flow path and the second flow path. The light source unit is disposed to face the lower end of the inner tube unit. The lower end of the inner tube unit is located within an irradiation area in which the fluid is exposed to light directly from the light source unit. A central axis of the inlet port lies on a different line than a central axis of the inner tube unit.

## Description

### [Technical Field]

The present invention relates to a fluid treatment device.

### [Background Art]

Recently, as pollution from industrialization has increased, environmental concerns have grown and the focus on wellness has intensified. As a result, there is growing demand for clean water and clean air, which is leading to development of various related products such as water purifiers and air purifiers capable of providing clean water and clean air.

### [Disclosure]

### [Technical Problem]

It is an aspect of the present invention to provide a fluid treatment device that can purify a fluid.

It is another aspect of the present invention to provide a fluid treatment device that can improve fluid treatment efficiency by minimizing optical loss.

It is a further aspect of the present invention to provide a fluid treatment device employing a method that can easily improve the yield of mass production.

It is a yet another aspect of the present invention to provide a fluid treatment device capable of reducing malfunction and damage caused by heat generation of a drive circuit.

### [Technical Solution]

In accordance with an embodiment of the present invention, there is provided a fluid treatment device including: a housing; an inner tube unit disposed inside the housing; and a light source unit emitting light to an inner space of the housing. The housing includes an inlet port through which a fluid is introduced and an outlet port through which the fluid is discharged. The inner tube unit has a smaller inner diameter than the housing and is open at a lower end thereof. An inner space of the inner tube unit defines a first flow path, and a space between the inner tube unit and the housing defines a second flow path. The fluid introduced through the inlet port flows to the outlet port after passing through the first flow path and the second flow path. The light source unit is disposed to face the lower end of the inner tube unit. The lower end of the inner tube unit is located within an irradiation area in which the fluid is exposed to light directly from the light source unit. A central axis of the inlet port lies on a different line than a central axis of the inner tube unit.

### [Advantageous Effects]

The fluid treatment device according to the embodiment of the present invention can purify a fluid by irradiating the fluid with sterilizing light.

In addition, the fluid treatment device according to the embodiment of the present invention can improve fluid treatment efficiency by minimizing light loss.

In addition, the fluid treatment device according to the embodiment of the present invention is easy to assemble and mass-produce, and can achieve enhanced yield rate and reduced defect rate.

In addition, the fluid treatment device according to the embodiment of the present invention can prevent a light source from malfunctioning or being damaged due to heat generated from a drive circuit by placing the drive circuit outside a housing.

### [Description of Drawings]

FIG. 1 is a front view of a fluid treatment device according to an embodiment of the present invention.
FIG. 2 is an exploded view of the fluid treatment device according to the embodiment of the present invention.
FIG. 3 is a cross-sectional view of the fluid treatment device according to the embodiment of the present invention.
FIG. 4 is a plan view of a first housing of the fluid treatment device according to the embodiment of the present invention.
FIG. 5 is a cross-sectional view of the first housing of the fluid treatment device according to the embodiment of the present invention.
FIG. 6 is a cross-sectional view illustrating another embodiment of a second seating unit and an inflow hole of the fluid treatment device according to the embodiment of the present invention.
FIG. 7 is a perspective view of a first reflector of the fluid treatment device according to the embodiment of the present invention.
FIG. 8 is a cross-sectional view of the first reflector of the fluid treatment device according to the embodiment of the present invention.
FIG. 9 is a perspective view of an inner tube unit of the fluid treatment device according to the embodiment of the present invention.
FIG. 10 is a cross-sectional view of the inner tube unit of the fluid treatment device according to the embodiment of the present invention.
FIG. 11 is a cross-sectional view illustrating another embodiment of the inner tube unit of the fluid treatment device according to the embodiment of the present invention.
FIG. 12 is a cross-sectional view illustrating a further embodiment of the inner tube unit of the fluid treatment device according to the embodiment of the present invention.
FIG. 13 is a cross-sectional view illustrating yet another embodiment of the inner tube unit of the fluid treatment device according to the embodiment of the present invention.
FIG. 14 is a plan view of a second housing according to the embodiment of the present invention.
FIG. 15 is a cross-sectional view of the second housing according to the embodiment of the present invention.
FIG. 16 is a perspective view of a second reflector according to the embodiment of the present invention.
FIG. 17 is a cross-sectional view of the second reflector according to the embodiment of the present invention.
FIG. 18 is a view illustrating another embodiment of the second reflector.
FIG. 19 is a plan view of the second housing of the fluid treatment device according to the embodiment of the present invention, with the light source unit and the second reflector disposed therein.
FIG. 20 is a cross-sectional view of the second housing of the fluid treatment device according to the embodiment, with the light source unit and the second reflector disposed therein.
FIG. 21 is an exemplary view illustrating the length of an inner tube of the fluid treatment device according to the embodiment of the present invention.
FIG. 22 is an exemplary view illustrating the length of the inner tube when the fluid treatment device according to the embodiment includes a plurality of light sources of the present invention.
FIG. 23 is another exemplary view illustrating the length of the inner tube when the fluid treatment device according to the embodiment includes a plurality of light sources of the present invention.
FIG. 24 is a view illustrating the location of an inlet port of the fluid treatment device according to the embodiment of the present invention.
FIG. 25 is a view illustrating an external appearance of another embodiment of the inlet port and the outlet port of the fluid treatment device according to the embodiment of the present invention.
FIG. 26 is a cross-sectional view of a further embodiment of the inlet port and the outlet port of the fluid treatment device according to the embodiment of the present invention.

### [Best Mode]

The following embodiments are provided as examples to enable those skilled in the art to fully understand the concept of the present invention. Therefore, the present invention is not limited to the embodiments described below and may be embodied in other forms. In addition, in the drawings, the width, length, thickness, and other dimensions of components may be exaggerated for convenience. Like components will be denoted by like reference numerals throughout the specification.

Hereinafter, embodiments of the present invention will be described with reference to the accompanying drawings.

A fluid treatment device according to an embodiment of the present invention includes: a housing; an inner tube unit disposed inside the housing; and a light source unit emitting light to an inner space of the housing. The housing includes an inlet port through which a fluid is introduced and an outlet port through which the fluid is discharged. The inner tube unit has a smaller inner diameter than the housing and is open at a lower end thereof. An inner space of the inner tube unit defines a first flow path, and a space between the inner tube unit and the housing defines a second flow path. The fluid introduced through the inlet port flows to the outlet port after passing through the first flow path and the second flow path. The light source unit is disposed to face the lower end of the inner tube unit. The lower end of the inner tube unit is located within an irradiation area in which the fluid is exposed to light directly from the light source unit. A central axis of the inlet port lies on a different line than a central axis of the inner tube unit.

The fluid treatment device may further include: a first reflector closely contacting an inner wall of the housing and spaced apart from the inner tube unit. The first reflector may include a material reflecting light from the light source unit.

The first reflector may be formed by injection molding and may be inserted into the housing.

The inner tube unit may include an inner tube defining the first flow path and formed of a light transmissive material.

The housing may include a stepped seating unit on which the inner tube unit is seated through insertion of an upper end of the inner tube unit. The seating unit may be formed with an inflow hole connected to the inlet port.

The seating unit may include a first step and a second step located outside the first step. The first step may have a lower height than the second step.

The upper end of the inner tube unit may be inserted into the second step of the seating unit to be seated on the first step.

The inflow hole may be formed on the first step of the seating unit.

The inflow hole may be formed on the first step of the seating unit to extend to an inner wall of the housing located inside the seating unit.

A diameter of the inflow hole may be smaller than an inner diameter of the inlet port.

The fluid treatment device may further include: a second reflector disposed between the inner tube unit and the light source unit inside the housing. A region of the light source unit may be placed inside the second reflector.

The second reflector may include a reflective member and a window member. The reflective member may be formed with a through-hole and may reflect light from the light source unit. The window member may disposed on an upper surface of the reflective member to cover the through-hole of the reflective member. **In** addition, the window member may be formed of a material transmitting light from the light source unit. A region of the light source unit may be disposed in the through-hole of the reflective member of the second reflector.

The second reflector may include a sealing member covering at least a region of a side surface of the reflective member of the second reflector, a region of an upper surface of the reflective member, and an upper edge of the window member.

Each of the inlet port and the outlet port may have a bent shape.

The irradiation area may include a concentrated sterilization zone having a sterilizing power of 90% or more against contaminants contained in the fluid.

The light source unit may include a plurality of light sources.

Here, the lower end of the inner tube unit may be located in a region where irradiation areas of at least two light sources overlap.

The housing may include: a first housing and a second housing. The inner tube unit may be disposed in the first housing. The second reflector and the light source unit may be disposed in the second housing. The first housing and the second housing may have different outer diameters. The first housing may be coupled to the second housing through insertion of a region of the first housing into the second housing.

The fluid treatment device may further include: a drive unit disposed outside the housing and including a drive circuit controlling operation of the light source unit.

Hereinafter, a fluid treatment device according to the present invention will be described in detail with reference to the accompanying drawings.

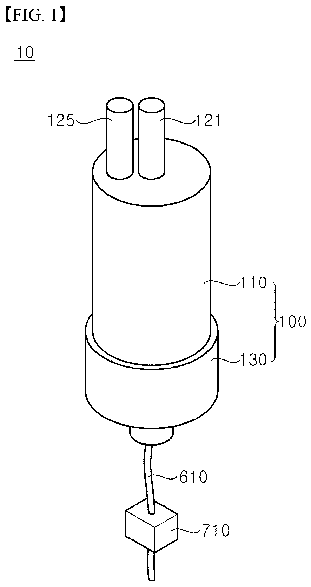

FIG. 1 to FIG. 26 are exemplary views illustrating a fluid treatment device according to embodiments of the present invention.

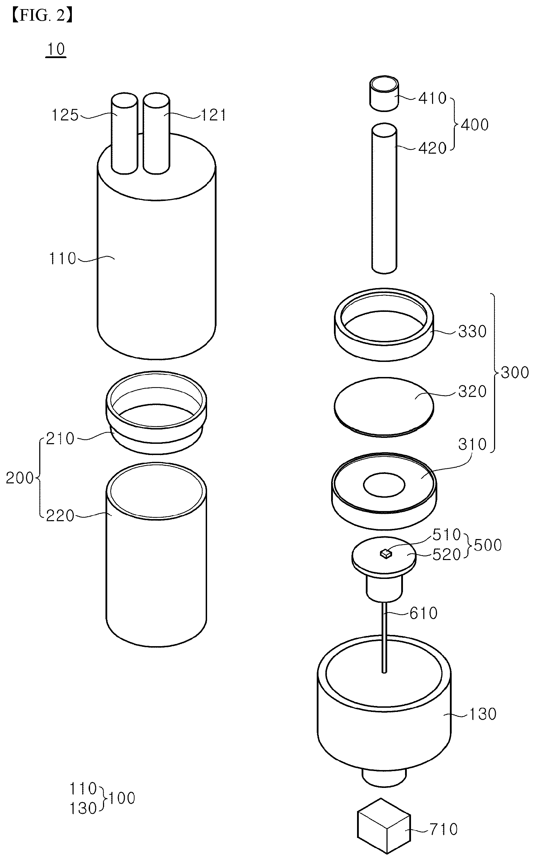

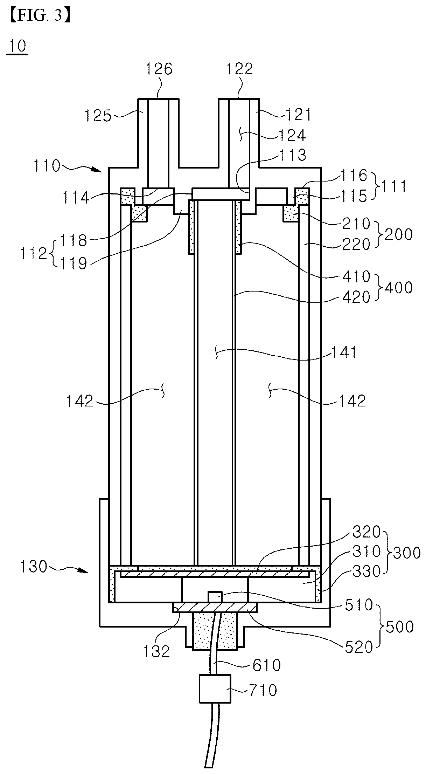

FIG. 1 is a front view of a fluid treatment device 10 according to an embodiment of the present invention. FIG. 2 is an exploded view of the fluid treatment device 10 according to the embodiment. FIG. 3 is a cross-sectional view of the fluid treatment device 10 according to the embodiment.

Referring FIG. 1 to FIG. 3, the fluid treatment device 10 according to this embodiment includes a housing 100, a first reflector 200, a second reflector 300, an inner tube unit 400, and a light source unit 500.

The fluid treatment device 10 is a device that sterilizes a fluid introduced therein. In this embodiment, fluid treatment includes sterilizing introduced raw water to produce purified water. That is, the fluid refers to water and fluid treatment refers to sterilization.

The housing 100 includes a first housing 110 and a second housing 130.

The first housing 110 and the second housing 130 are coupled to each other to form an inner space in which various components for sterilization of raw water are disposed to sterilize introduced raw water.

The first housing 110 is formed on an upper surface thereof with an inlet port 121, which is a passage through which raw water is introduced into the first housing, and an outlet port 125, which is a passage through which purified water is discharged from the first housing. In addition, the first housing 110 is formed on a lower surface thereof with a hole connected to an inner space of the first housing 110.

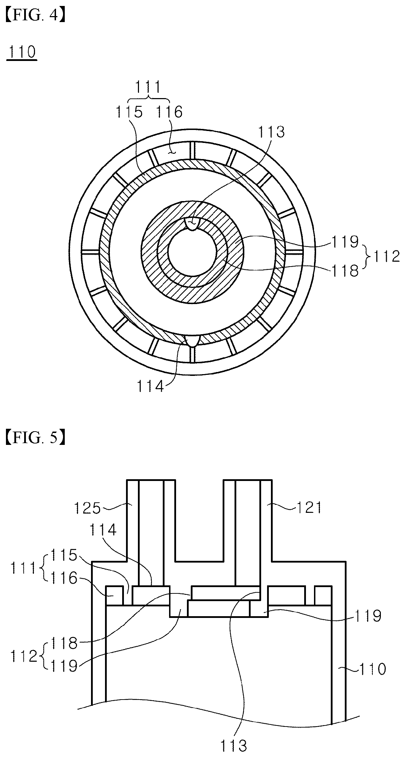

FIG. 4 is a plan view of the first housing 110 of the fluid treatment device 10 according to the embodiment. FIG. 5 is a cross-sectional view of the first housing 110 of the fluid treatment device 10 according to the embodiment.

Referring to FIG. 3 and FIG. 4, the first housing 110 is formed therein with a first seating unit 111, a second seating unit 112, an inflow hole 113, and a discharge hole 114.

The first seating unit 111 allows the first reflector 200 to be seated thereon.

The first seating unit 111 includes a first protrusion 115 protruding downwards from an inner upper surface of the first housing 110. The first projection 115 may be formed along an inner side surface of the first housing 110 with a space therebetween. Thus, a first groove 116 having a concave shape with respect to an upper surface of the first protrusion 115 may be formed between the first protrusion 115 and the inner side surface of the first housing 110. The first protrusion 115 and the first groove 116 may define the first seating unit 111 on which one end of the first reflector 200 is seated. Although the first protrusion 115 is shown as being formed in a circular shape along the inner side surface of the first housing 110 in this embodiment, the shape of the first protrusion 115 is not limited thereto. The first protrusion 115 may correspond in shape to one end of the first reflector 200 that is seated on the first seating unit 111. However, the present invention is not limited thereto and the first protrusion 115 may have various other shapes so long as the first reflector 200 can be seated thereon.

The second seating unit 112 allows the inner tube unit 400 to be seated thereon.

The second seating unit 112 may be formed inside the first seating unit 111 and may be spaced apart from the first seating unit 111. In addition, the second seating unit 112 may also be formed in a circular shape along the first seating unit 111. Further, the second seating unit 112 may have a stepped structure protruding downwards from the inner upper surface of the first housing 110. In this embodiment, the stepped structure of the second seating unit 112 may have a first step 118 and a second step 119 formed outside the first step, wherein the first step 118 has a lower height than the second step 119. One end of the inner tube unit 400 may be seated on an inner side surface of the stepped structure of the second seating unit 112. Although the second seating unit 112 is shown as having a circular cross-section in this embodiment, the present invention is not limited thereto. The second seating unit 112 may correspond in shape to the one end of the inner tube unit 400. However, the present invention is not limited thereto and the second seating unit 112 may have various other shapes so long as the one end of the inner tube unit 400 can be seated thereon.

The inflow hole 113 may be formed on the second seating unit 112. Here, at least a region of the inflow hole 113 may be formed on a side surface of the second seating unit 112. More specifically, the inflow hole 113 may be formed on the first step 118 of the second seating unit 112. Alternatively, the inflow hole 113 may extend from the first step 118 of the second seating unit 112 to be further formed on an inner upper surface of the first housing 110 located inside the second seating unit 112.

Referring to FIG. 5, the inflow hole 113 may be formed in side surfaces of the first step 118 and the second step 119 of the first seating unit 111 and may have a constant diameter. However, the shape of the inflow hole 113 is not limited thereto.

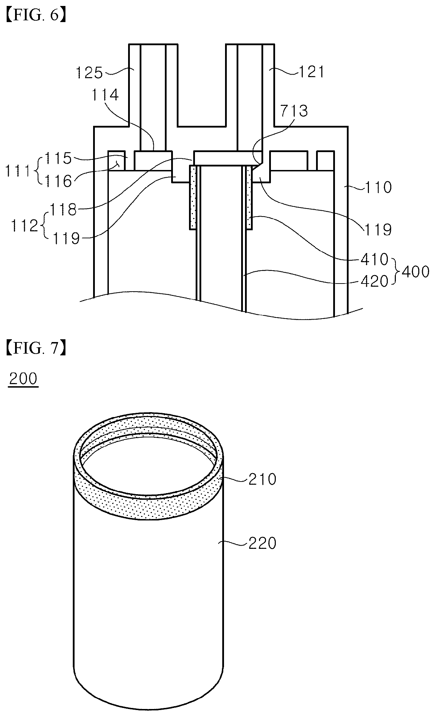

FIG. 6 is a cross-sectional view illustrating another embodiment of a second seating unit 712 and an inflow hole 713 of the fluid treatment device 10 according to the embodiment of the present invention.

Referring to FIG. 6, the inflow hole 713 may be gradually increased in diameter from an outer side surface of the second seating unit 712 toward an inner side surface of the second seating unit 712. Here, an inner wall of the second seating unit 712 defining the inflow hole 713 and located adjacent to an inner tube 420 may be inclined.

When a fluid collides with a corner of a flow path in the process of changing direction, a vortex is generated, causing fluid resistance.

Due to the inclined inner wall, the inflow hole 713 according to this embodiment can minimize collision with the fluid as the fluid changes direction. In addition, since the inflow hole 713 according to this embodiment is gradually increased in diameter in a moving direction of a fluid, it is possible to prevent vortex-induced stagnation of the fluid within or near the inflow hole 713.

Although the second seating unit 712 according to this embodiment has a structure in which an inner wall of the region thereof defining the inflow hole 713 is inclined such that the inflow hole 713 is gradually increased in diameter, as shown in FIG. 6, the structure of the second seating unit 112 is not limited thereto. For example, the second seating unit 112 may be formed such that the entirety of the inner wall thereof, rather than a region of the inner wall, is inclined.

Referring to FIG. 3 and FIG. 4, the inflow hole 113 formed on the second seating unit 112 may be connected to an inlet passage 124 of the inlet port 121 of the first housing 110. Here, the inlet passage 124 is an inner space of the inlet port 121 through which a fluid flows. Accordingly, raw water introduced through the inlet port 121 may be introduced into the housing 100 through the inflow hole 113.

The discharge hole 114 may be formed on an inner upper surface of the first housing 110 located inside the first seating unit 111. More specifically, the discharge hole 114 may be formed on an inner upper surface of the first housing 110 located between the first seating unit 111 and the second seating unit 112.

The discharge hole 114 may be connected to an outlet passage of the outlet port 125 of the first housing 110. Accordingly, raw water sterilized inside the housing 100, that is, purified water, may be discharged to the outlet port 125 through the discharge hole 114.

The first reflector 200 is disposed inside the first housing 110.

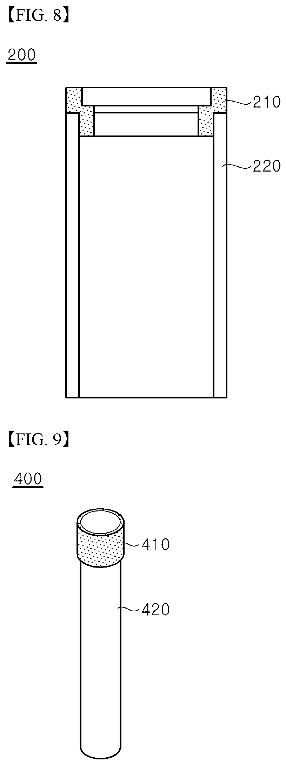

FIG. 7 is a perspective view of the first reflector 200 of the fluid treatment device 10 according to the embodiment. FIG. 8 is a cross-sectional view of the first reflector 200 of the fluid treatment device 10 according to the embodiment.

Referring to FIG. 2, FIG. 3, FIG. 7, and FIG. 8, the first reflector 200 may include a first sealing member 210 and a first reflective member 220.

The first reflective member 220 may have a cylindrical shape having an inner space. In addition, the first reflective member 220 may be disposed inside the first housing 110.

The first sealing member 210 may be disposed at one end of the first reflective member 220 to be coupled to the first reflective member 220.

The first sealing member 210 may be formed with a through hole extending from an upper surface thereof to a lower surface thereof and may have a stepped outer side surface. That is, the outer side surface of the first sealing member 210 may be formed with a step structure. An upper surface of the first reflective member 220, that is, the one end of the first reflective member 220, may be seated on the step of the outer side surface of the first sealing member 210.

The first sealing member 210 may be divided into an upper region and a lower region having different diameters. For example, the upper region of the first sealing member 210 may have a larger outer diameter than the lower region of the first sealing member 210. In addition, the upper region of the first sealing member 210 may have a larger inner diameter than the lower region of the first sealing member 210. The first sealing member 210 may be coupled to the first reflective member 220 in such a way that the upper region of the first sealing member 210 is placed at an upper region of the first reflective member 220 and the lower region of the first sealing member 210 is inserted into the first reflective member 220. In addition, the upper region of the first sealing member 210 may have the same outer diameter as the first reflective member 220. Further, the outer diameter of the lower region of the first sealing member 210 may be the same as the inner diameter of the first reflective member 220. Accordingly, when the first sealing member 210 is coupled to the first reflective member 220, the lower region of the first sealing member 210 may closely contact an inner side surface of the first reflective member 220 and the upper region of the first sealing member 210 may closely contact an upper surface of the first reflective member 220. As such, since the first sealing member 210 closely contact the inner side surface and the upper surface of the first reflective member 220, a fluid flowing in the inner space of the first reflector 200 can be prevented from leaking through a gap between the first reflective member 220 and the first sealing member 210.

Referring to FIG. 8, the lower region of the first sealing member 210 may have a smaller inner diameter than the first reflective member 220. However, the shape of the first sealing member 210 is not limited thereto. For example, both the upper region and the lower region of the first sealing member 210 may have the same inner diameter as the first reflective member 220.

The first sealing member 210 may be formed of an elastic material. Accordingly, when the first housing 110 is coupled to the second housing 130, the upper surface of the first sealing member 210 can be firmly held against the first housing 110 and the lower surface of the first sealing member 210 can be firmly held against the first reflective member 220 due to elasticity of the first sealing member 210. As a result, watertightness between the first housing 110, the first sealing member 210, and the first reflective member 220 can be enhanced.

The first reflective member 220 may be formed of a reflective material. Since the first reflective member 220 is formed of a reflective material, light emitted from the light source unit 500 may be reflected from an inner wall of the first reflective member 220 without being absorbed thereby. Accordingly, light emitted from the light source unit 500 can be prevented from being lost by the first reflective member 220. Furthermore, a fluid flowing through the inner space of the first reflective member 220 can be exposed not only to light directly from the light source unit 500 but also to light reflected from the first reflective member 220. Accordingly, the first reflective member 220 can ensure increase in amount of light to which the fluid is exposed or reduction in light loss, thereby improving fluid treatment efficiency. For example, the first reflective member 220 may be formed of Teflon.

The fluid treatment device 10 according to this embodiment includes the first reflective member 220 formed of a reflective material as a separate component, instead of coating the first housing 110 with a reflective material.

To coat the inner wall of the first housing 110 with a reflective material such as Teflon, the reflective material needs to be formed into a liquid resin before being applied to the inner wall of the first housing 110. As such, the first housing 110 coated with a reflective material on an inner wall thereof is formed through a complex multi-step process, making mass production difficult.

In contrast, in the fluid treatment device 10 according to this embodiment, the first reflective member 220 is fabricated by injection molding and is inserted into the first housing 110. By adopting a simple method of forming the first reflective member 220 through injection molding and inserting the first reflective member into the first housing 110, it is possible to ensure that reflected light reaches the entire inner space of the first housing 110. This method of providing the first reflective member 220 as a separate component and assembling the first reflective member into the first housing 110 is more suitable for mass production than coating the inner wall of the first housing 110 with a reflective material.

Referring to FIG. 3, the upper region of the first sealing member 210 may be at least partially inserted into the first seating unit 111. That is, the first reflector 200 is secured to the first housing 110 by inserting the upper region of the first sealing member 210 between the first protrusion 115 and the inner side surface of the first housing 110. When the first reflector 200 is seated on the first seating unit 111 of the first housing 110, the outer side surface of the first reflective member 220 may closely contact the inner side surface of the first housing 110.

In addition, the first sealing member 210 may seal the first seating unit 111 of the first housing 110. That is, the first sealing member 210 can prevent a fluid from entering a space between the first protrusion 115 and the inner side surface of the first housing 110. If a fluid enters the space between the first protrusion 115 of the first housing 110 and the inner side surface of the first housing 110 and stays in the space for a long period of time, the fluid can be further contaminated. The first sealing member 210 prevents a fluid from entering a space between the first protrusion 115 and the first housing 110, thereby preventing the fluid from stagnating in this space and becoming contaminated.

When the first reflector 200 is coupled to the first housing 110, the discharge hole 114 is placed inside the first reflector 200. That is, the inner space of the first reflector 200 may be connected to the discharge hole 114. Accordingly, a fluid flowing through the inner space of the first reflector 200 can be discharged to the outlet port 125 through the discharge hole 114.

The inner tube unit 400 is disposed inside the first reflector 200.

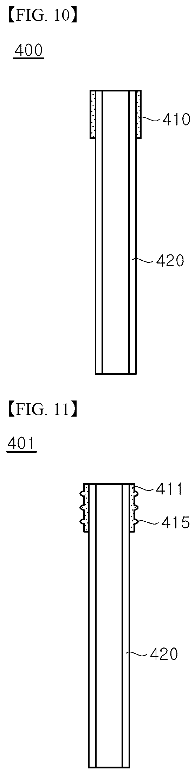

FIG. 9 is a perspective view of the inner tube unit 400 of the fluid treatment device 10 according to the embodiment. FIG. 10 is a cross-sectional view of the inner tube unit 400 of the fluid treatment device 10 according to the embodiment.

Referring to FIG. 2, FIG. 3, FIG. 9, and FIG. 10, the inner tube unit 400 may include a second sealing member 410 and an inner tube 420.

The second sealing member 410 may be formed with a through-hole extending from an upper surface thereof to a lower surface thereof. An upper region of the inner tube 420, which is one end of the inner tube 420, may be inserted into the through-hole of the second sealing member 410. That is, the second sealing member 410 may be formed to cover the upper region of the inner tube 420.

Referring to FIG. 10, the second sealing member 410 may be formed to cover an upper side surface of the inner tube 420. Accordingly, when the second sealing member 410 is mounted on the inner tube 420, an inner diameter of the second sealing member 410 may be the same as an outer diameter of the inner tube 420. In addition, the second sealing member 410 may be formed of an elastic material.

The inner tube 420 may be a cylindrical structure formed with a through-hole extending from an upper surface thereof to a lower surface thereof. In addition, the inner tube 420 may be formed of a material that transmits light from the light source unit 500. Accordingly, light reflected from the first reflective member 220 can pass through the inner tube 420 to irradiate the inner space of the inner tube 420. Additionally, light emitted from the light source unit 500 and having reached the inner space of the inner tube 420 can pass through the inner tube 420 to irradiate a space between the inner tube 420 and the first reflective member 220. For example, the inner tube 420 may be formed of quartz.

The inner tube unit 400 may be secured to the first housing 110 as the second sealing member 410 covering the inner tube 420 is inserted into and seated on the second seating unit 112 of the first housing 110.

Here, an upper surface of the second sealing member 410 may closely contact a surface of the first step 118 of the second seating unit 112. Here, the second sealing member 410 may have a smaller inner diameter than the first step 118 of the second seating unit 112. Accordingly, an inner edge of the first step 118 of the second seating unit 112 may be placed on the upper surface of the second sealing member 410, rather than on the upper surface of the inner tube 420. If the first step 118 of the second seating unit 112 closely contacts the inner tube 420, the inner tube 420 can be damaged by the second seating unit 112 due to the force applied to insert the inner tube unit 400 into the second seating unit 112. According to this embodiment, since the first step 118 of the second seating unit 112 closely contacts the second sealing member 410 having elasticity, rather than the inner tube 420, it is possible to prevent the inner tube 420 from being damaged by the second seating unit 112.

When the inner tube unit 400 is inserted into the second seating unit 112, a side surface of the second sealing member 410 may closely contact an inner side surface of the second step. Accordingly, when the inner tube unit 400 is seated on the second seating unit 112 of the first housing 110, an outer diameter of the second sealing member 410 may be the same as an inner diameter of the upper end of the second seating unit 112.

Referring to FIG. 3, the inner tube unit 400 is placed under the first step 118 of the second seating unit 112 and thus does not block the inflow hole 113 formed on the second seating unit 112. Accordingly, when the inner tube unit 400 is coupled to the second seating unit 112, the inflow hole 113 is connected to the inner space of the first step 118 of the second seating unit 112 and the inner space of the first step 118 is connected to the inner space of the inner tube 420. Accordingly, a fluid introduced through the inlet port 121 can be introduced into the inner space of the inner tube 420 by sequentially passing through the inflow hole 113 and the inner space of the first step 118 of the second seating unit 112.

Additionally, when the inner tube unit 400 is coupled to the second seating unit 112, the lower surface of the inner tube unit 400, which is the other end of the inner tube unit 400, may be placed adjacent to a window member 320 of the second reflector 300 with a space therebetween. Since the inner tube unit 400 is spaced apart from the second reflector 300, a fluid in the inner space of the inner tube 420 can move to a space between the first reflective member 220 and the inner tube 420 through the space between the inner tube 420 and the second reflector 300.

In typical methods, a through-hole as a fluid passage is formed through a region of the inner tube 420 (for example, a sidewall of the inner tube 420) to move a fluid in the inner tube 420 from the inside of the inner tube 420 to the outside of the inner tube 420. However, when the inner tube 420 is formed of a rigid material such as quartz, the inner tube 420 can be damaged during formation of a through-hole through the sidewall of the inner tube 420. In particular, when the sidewall of the inner tube 420 has a curved shape, the probability of damage to the inner tube during formation of the through-hole increases. In contrast, the fluid treatment device 10 according to this embodiment includes an inner tube 420 open at a bottom (lower end) thereof, instead of an inner tube 420 with a fluid passage formed through a sidewall thereof. In addition, in the fluid treatment device 10, the inner tube 420 is positioned such that the open bottom thereof is spaced apart from the second reflector 300, whereby a space for passage of a fluid is formed between the inner tube 420 and the second reflector 300. Accordingly, the fluid treatment device 10 according to the present embodiment can eliminate the need for the process of forming a fluid passage through the sidewall of the inner tube 420, thereby reducing the risk of damage to the inner tube 420 and thus enhancing the yield of the inner tube 420. Furthermore, this feature can ensure increase in yield of the fluid treatment device 10 and reduction in defect rate.

Although not shown in FIG. 10, an adhesive material may be interposed between the second sealing member 410 and the inner tube 420. Accordingly, adhesion between the second sealing member 410 and the inner tube 420 is enhanced, thereby preventing the second sealing member 410 from being separated from the inner tube 420 due to various factors such as hydraulic pressure in the inner and outer spaces of the inner tube 420, external impact, and the like.

Referring to FIG. 9 and FIG. 10, the inner tube unit 400 has a structure in which the second sealing member 410 covers only the upper side surface of the inner tube 420. However, the structure of the inner tube unit 400 is not limited thereto.

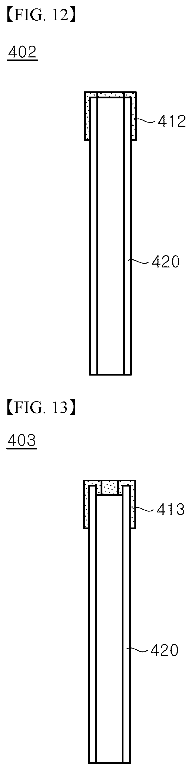

FIG. 11 is a cross-sectional view illustrating another embodiment of the inner tube unit of the fluid treatment device 10 according to the embodiment of the present invention. FIG. 12 is a cross-sectional view illustrating a further embodiment of the inner tube unit of the fluid treatment device 10 according to the embodiment of the present invention. FIG. 13 is a cross-sectional view illustrating yet another embodiment of the inner tube unit of the fluid treatment device 10 according to the embodiment of the present invention.

Referring to FIG. 11, an inner tube unit 401 may include a second sealing member 411 having an outer side surface with at least a projection 415 formed thereon. The projection 415 may be formed of the same material as the second sealing member 411. Accordingly, the projection 415 has elasticity, like the second sealing member 411.

The projection 415 of the second sealing member 411 ensures that the inner tube 401 is more firmly secured to the second seating unit 112 of the first housing 110 upon insertion of the inner tube 401 into the second seating unit 112. Accordingly, it is possible to prevent separation of the inner tube 401 from the second seating unit 112 due to the pressure of a fluid introduced into the first flow path 141 of the inner tube 420.

Referring to FIG. 12, a second sealing member 412 of an inner tube unit 402 may cover not only the upper side surface of the inner tube 420, but also the upper surface of the inner tube 420. Referring to FIG. 13, a second sealing member 413 of an inner tube unit 403 may cover the upper side surface of the inner tube 420, the upper surface of the inner tube 420, and a region of the inner side surface of the inner tube 420.

The second sealing member 412; 413 (see FIG. 12 and FIG. 13) has a larger contact area with the inner tube 420 than the second sealing member 410 (see FIG. 9 and FIG. 10). Accordingly, the second sealing member 412; 413 (see FIG. 12 and FIG. 13) can have enhanced adhesion to the inner tube 420 due to the increased contact area with the inner tube 420.

Further, since the second sealing member 412; 413 covers the upper surface of the inner tube 420, the upper surface of the second sealing member 412; 413 is placed between the inner tube 420 and the second seating unit 112. Accordingly, the second sealing member 412; 413 can prevent the inner tube 420 from being damaged due to collision with the second seating unit 112 or due to the force exerted on the inner tube 420 in the direction of the second seating unit 112.

Referring to FIG. 3, the second housing 130 coupled to the first housing 110 has a larger diameter than the first housing 110. For example, an outer diameter of the second housing 130 may be larger than an outer diameter of the first housing 110. In addition, an inner diameter of the second housing 130 may be the same as the outer diameter of the first housing 110. Accordingly, the first housing 110 and the second housing 130 may be coupled to each other by inserting a lower region of the first housing 110 into the second housing 130.

For example, the first housing 110 and the second housing 130 may be coupled to each other by thread fastening. Specifically, the first housing 110 may be threaded on a lower outer side surface thereof, and the second housing 130 may be threaded on an inner side surface thereof. Accordingly, the first housing 110 and the second housing 130 may be coupled to each other by fastening the first housing 110 to the second housing 130 such that the male and female threads of the first housing 110 are engaged with the female and male threads of the second housing 130, respectively.

Although it is described that the first housing 110 and the second housing 130 are formed separately from each other and detachably coupled to each other in this embodiment, the present invention is not limited thereto and the first housing 110 and the second housing 130 may be integrally formed with each other.

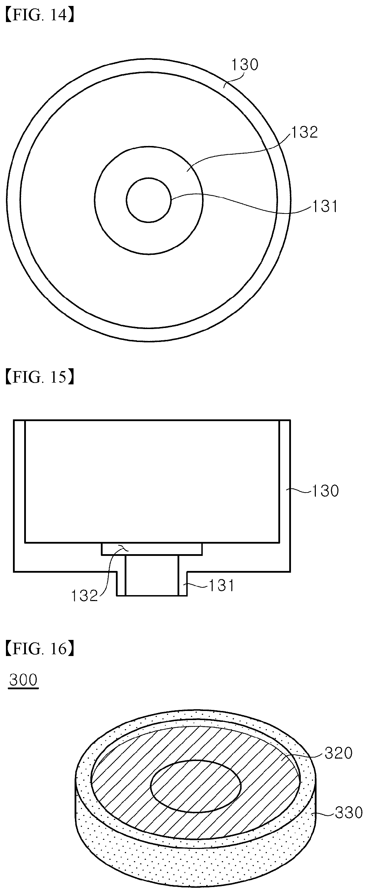

FIG. 14 is a plan view of the second housing 130 according to the embodiment of the present invention. FIG. 15 is a cross-sectional view of the second housing 130 according to the embodiment of the present invention.

The light source unit 500 and the second reflector 300 may be disposed in an inner space of the second housing 130.

Referring to FIG. 14 and FIG. 15, the second housing 130 is open at a top thereof and has an inner space. As shown in FIG. 3, the first housing 110 may be inserted into the inner space of the second housing 130 through the open top of the second housing 130.

In addition, the second housing 130 may be formed on a lower surface thereof with a second protrusion 131 protruding downwards. The second protrusion 131 is formed with a through-hole through which an electric wire 610 connecting the light source unit 500 to an external power source passes. That is, the through-hole formed through the second protrusion 131 may connect the inner space of the second housing 130 to an exterior of the second housing 130.

The second housing 130 may include a third seating unit 132 formed on an inner lower surface thereof. Referring to FIG. 15, the third seating unit 132 may protrude upwards from the inner lower surface of the second housing 130. Referring to FIG. 14, the third seating unit 132 may extend along an edge of an inner side surface of the second housing 130 with a space between the third seating unit 132 and the inner side surface of the second housing 130. The light source unit 500 is mounted in an inner region of the third seating unit 132.

Referring to FIG. 14, the third seating unit 132 may have a circular edge. However, the structure of the third seating unit 132 is not limited thereto and the third seating unit 132 may have any shape so long as the light source unit 500 can be mounted inside the third seating unit.

The third seating unit 132 may secure the light source unit 500 such that the light source unit 500 mounted in the inner region thereof can be prevented from being moved in a horizontal direction. To this end, the third seating unit 132 may have a shape and size that allows both sides of the light source unit 500 to at least partially closely contact the inner side surface of the third seating unit 132. For example, the third seating unit 132 may have an inner side surface corresponding in shape to an outer side surface of a substrate 520 of the light source unit 500 and may have an inner diameter identical to an outer diameter of the substrate 520 of the light source unit 500. Accordingly, when the light source unit 500 is mounted inside the third seating unit 132, the inner side surface of the third seating unit 132 may closely contact the outer side surface of the substrate 520 of the light source unit 500.

Referring to FIG. 2, the light source unit 500 may include at least a light source 510 and a substrate 520 on which the light source 510 is mounted.

According to this embodiment, the light source 510 may emit light capable of sterilizing contaminants contained in a fluid. For example, the light source 510 may emit UV light. Further, the light source 510 may emit UVC. Further, the light source 510 may emit light having a peak wavelength in the wavelength range of about 265 nm to about 275 nm.

The substrate 520 may serve to electrically connect the light source 510 to an external power source. Accordingly, power from the external power source may be supplied to the light source 510 through the substrate 520.

The substrate 520 may include a base, an upper pattern, a bottom pattern, and a via. The base may be formed of an insulating material. The upper pattern may be formed on an upper surface of the base, and the lower pattern may be formed on a lower surface of the base. Both the upper pattern and the lower pattern may be formed of a conductive material. For example, the upper pattern and the lower pattern may be formed of copper. The via may be formed to pass through the base and may have one end contacting the upper pattern and the other end contacting the lower pattern. Here, the via may be formed of a conductive material such as copper, solder paste, and the like.

According to this embodiment, the upper pattern of the substrate 520 may be electrically connected to the light source 510, the lower pattern of the substrate 520 may be electrically connected to an electric wire 610 connected to the external power source, and the via may electrically connect the upper pattern to the lower pattern. Accordingly, power from the external power source may be supplied to the light source 510 through the substrate 520. The upper pattern, the lower pattern, and the via constitute a conductive interconnect.

Here, the electric wire 610 may pass through the through-hole of the second protrusion 131 of the second housing 130 to connect the light source unit 500 to the external power source.

Referring to FIG. 1, the fluid treatment device 10 according to this embodiment may include a drive unit 710 disposed outside the housing 100. The drive unit 710 may include a drive circuit to drive the light source 510 of the light source unit 500 at a constant voltage or at a constant current. That is, the drive circuit may be a circuit that controls operation of the light source unit. To implement the drive circuit, a large-area substrate and electronic components are required. Moreover, the drive circuit generates high heat during operation.

Since the fluid treatment device 10 according to this embodiment includes the drive unit 710 including the drive circuit as a separate component, rather than implementing a drive circuit on the substrate 520 of the light source unit 500, the substrate 520 of the light source unit 500 can be manufactured in a compact size. Furthermore, in the fluid treatment device 10, the drive unit 710 including the drive circuit is disposed outside of the housing 100, thereby preventing the light source unit 500 from malfunctioning or being damaged due to heat from the drive circuit.

The drive unit 710 may further include a fault detector to detect a fault in the light source unit 500. The fault detector may detect abnormal conditions, such as short-circuit fault or an open-circuit fault in the light source 510 and may generate an abnormality detection signal to inform of the presence of abnormal conditions. For example, the fault detector may detect abnormal conditions of the light source 510 through detection of current or voltage flowing in the light source 510 and may generate an abnormality detection signal. Here, the fluid treatment device 10 may include an output unit configured to output sound, light, text, or the like in response to the abnormality detection signal from the fault detector. Upon receiving the abnormality detection signal from the fault detector through a wired or wireless connection, the output unit may output at least one of sound, text, or visible light to the exterior of the fluid treatment device 10 such that a user can confirm the presence of abnormal conditions visually or audibly. For example, the output unit may include a screen or a visible light source placed outside the fluid treatment device 10. Alternatively, the output unit may include a speaker placed inside or outside the fluid treatment device 10.

With the drive unit including the fault defector, the fluid treatment device 10 according to this embodiment ensures prompt replacement of the light source unit 500 when the light source unit 500 malfunctions, thereby preventing reduction in fluid treatment efficiency.

The second reflector 300 may be disposed above the light source unit 500. Here, the light source unit 500 may be partially placed inside the second reflector 300.

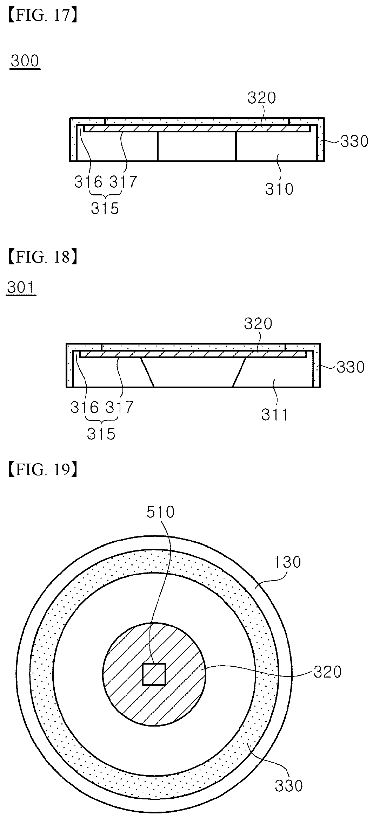

FIG. 16 is a perspective view of the second reflector 300 according to the embodiment of the present invention. FIG. 17 is a cross-sectional view of the second reflector 300 according to the embodiment of the present invention.

Referring to FIG. 2, FIG. 16, and FIG. 17, the second reflector 300 may include a second reflective member 310, a window member 320, and a fourth sealing member 330.

Referring to FIG. 17, the second reflective member 310 may include a through-hole extending from an upper surface thereof to a lower surface thereof. An outer diameter or cross-sectional area of the second reflective member 310 may be larger than the diameter or cross-sectional area of the substrate 520 of the light source unit 500. In addition, an outer diameter or cross-sectional area of the through-hole of the second reflective member 310 may be smaller than the diameter or cross-sectional area of the substrate 520 of the light source unit 500. Accordingly, when the second reflective member 310 is disposed above the light source unit 500, a lower surface of the second reflective member 310 is partially placed on an upper surface of the substrate 520 of the light source unit 500, as shown in FIG. 3. In addition, the light source 510 of the light source unit 500 is placed in the through-hole of the second reflective member 310.

According to this embodiment, the lower surface of the second reflective member 310 may have a flat shape without any steps. In addition, the lower surface of the second reflective member 310 may closely contact the upper surface of the substrate 520 of the light source unit 500.

The second reflective member 310 may be formed on an upper surface thereof with a fourth seating unit 315 on which the window member 320 is seated.

The second reflective member 310 includes a third protrusion 316 protruding upwards along an edge of the upper surface thereof. Accordingly, the upper surface of the second reflective member 310 includes a groove 317 recessed with respect to the third protrusion 316. The fourth seating unit 315 includes the third protrusion 316 and the groove 317 defined by the third protrusion 316. That is, the upper surface of the second reflective member 310 having a space allowing insertion of the window member 320 may form the fourth seating unit 315. An inner diameter of the fourth seating unit 315 is larger than the diameter of the through-hole of the second reflective member. The window member 320 may be inserted inside the third protrusion 316 of the second reflective member 310 to be seated on the fourth seating unit 315. The window member 320 may be formed of a material that transmits light emitted from the light source unit 500.

The fourth seating unit 315 may correspond in shape to the window member 320. For example, the fourth seating unit 315 may be formed such that an inner side surface thereof closely contact an outer side surface of the window member 320 while surrounding the outer side surface of the window member 320. Here, the inner side surface of the fourth seating unit 315 is an inner side surface of the third protrusion 316. Accordingly, the fourth seating unit 315 can prevent the window member 320 mounted on the upper surface of the second reflective member 310 from being displaced from a designated position thereof.

The second reflective member 310 may be formed of a material that reflects light emitted from the light source unit 500. Light from the light source unit 500 not directed at the window member 320 may be reflected by an inner side surface of the second reflective member 310 toward the window member 320. Accordingly, the second reflective member 310 ensures that as much light emitted from the light source unit 500 as possible passes through the window member 320 to irradiate the inner space of the fluid treatment device 10. By ensuring that as much light from the light source unit 500 as possible irradiates a fluid, the second reflective member 310 can improve fluid treatment efficiency of the fluid treatment device 10. For example, the second reflective member 310 may be formed of white silicone or Teflon. Alternatively, the second reflective member 310 may be formed of a metal. Since metals have high thermal conductivity, heat generated by the light source unit 500 can be dissipated to the outside of the light source unit 500 through the second reflective member 310. For example, the second reflective member 310 may be formed of aluminum.

When a fluid, such as a liquid or a gas, flows in the inner space of the housing 100, heat generated by the light source unit 500 can be transferred to the fluid through the second reflective member 310. For example, if the fluid is at a relatively low temperature compared to heat generated by the light source unit 500, the fluid can absorb the heat conducted to the second reflective member 310 before being discharged to the outside of the fluid treatment device 10. As a result, heat dissipation efficiency of the fluid treatment device 10 can be improved.

As such, with the second reflective member 310, the fluid treatment device 10 according to this embodiment can improve both light reflection and heat dissipation.

Alternatively, the second reflective member 310 may be formed by coating a reflective material on an inner wall thereof defining the through-hole, instead of forming a body thereof with a reflective material.

The second reflective member 310 may have a larger thickness than the light source 510 of the light source unit 500. Here, "thickness" refers to a distance between an upper surface and a lower surface of a corresponding component. Accordingly, when the light source 510 is disposed in the through-hole of the second reflective member 310, the light source 510 may be spaced apart from the window member 320.

An area irradiated with light emitted from the light source 510 increases with increasing distance from the light source 510 depending on the beam angle of the light source. Accordingly, a light incident area of the window member 320 decreases with decreasing distance between the light source 510 and the window member 320. Conversely, the light incident area of the window member 320 increases with increasing distance between the light source 510 and the window member 320. In addition, light can be diffused within the window member 320 while passing through the window member 320.

According to this embodiment, since the light source 510 and the window member 320 are separated from each other by the second reflective member 310, the light incident area of the window member 320 can be increased. In addition, light incident on the window member 320 over a larger area can be diffused more widely while passing through the window member 320 before entering the inner space of the housing 100. Accordingly, as much of the inner space of the housing 100 as possible can be exposed to the light transmitted through the window member 320.

Referring to FIG. 17, the second reflective member 310 has a structure in which the inner wall thereof defining the through-hole is perpendicular to the lower surface thereof. That is, the through-hole of the second reflective member 310 has a constant diameter from top to bottom. However, the structure of the second reflective member 310 is not limited thereto.

FIG. 18 is a view illustrating a second reflector 301 according to another embodiment. Referring to FIG. 18, a second reflective member 311 of the second reflector 301 has a structure in which an inner wall thereof defining a through-hole is inclined with respect to a lower surface thereof. The second reflective member 311 may include a through-hole gradually increased in diameter from bottom to top in consideration of the beam angle of the light source 510. That is, the second reflector 301 may include a second reflective member 311 including a through-hole gradually increased in diameter in the direction of travel of light emitted from the light source 510.

The window member 320 may be disposed on the fourth seating unit 315 of the second reflective member 310 to cover the through-hole of the second reflective member 310. The window member 320 may be formed of a material that transmits light emitted from the light source unit 500. For example, the window member 320 may be formed of quartz.

The window member 320 seated on the second reflective member 310 may cover the through-hole to prevent a fluid flowing in the inner space of the first housing 110 from entering the through-hole of the second reflective member 310.

The fourth sealing member 330 may be formed of an elastic material and may be formed with a through-hole extending from an upper surface thereof to a lower surface thereof. The second reflective member 310 may be securely inserted into an inner space of the fourth sealing member 330, that is, the through-hole of the fourth sealing member 330.

Referring to FIG. 17, the fourth sealing member 330 may be formed such that an upper through-hole 331 thereof has a smaller diameter than a lower through-hole 332 thereof. Accordingly, when the second reflective member 310 is inserted into the inner space of the fourth sealing member 330, the fourth sealing member 330 may cover a region of the upper surface of the second reflective member 310 and at least a region of the side surface of the second reflective member 310.

More specifically, the fourth sealing member 330 may be formed such that an upper region thereof closely covers a region of the upper surface of the second reflective member 310. In addition, the fourth sealing member 330 may be formed such that a lower region thereof closely covers the outer side surface of the second reflective member 310. Here, the upper region of the fourth sealing member 330 may cover at least a region of the fourth seating unit 315 of the second reflective member 310. Further, the upper region of the fourth sealing member 330 may cover the third protrusion 316 of the second reflective member 310 and a region of the upper surface of the window member 320 seated on the fourth seating unit 315, including an upper edge of the window member 320. Accordingly, the fourth sealing member 330 can closely secure the window member 320 to the second reflective member 310 by covering both the fourth seating unit 315 and the window member 320. The upper region of the fourth sealing member 330 corresponds to the upper surface of the second sealing member 410, and the lower region of the fourth sealing member 330 corresponds to the side and lower surfaces of the fourth sealing member 330, excluding the upper surface of the fourth sealing member.

The diameter of the upper through-hole 331 of the fourth sealing member 330 is larger than the diameter of the through-hole of the second reflective member 310 and is smaller than the inner diameter of the fourth seating unit 315 or the diameter of the window member 320. Accordingly, the fourth sealing member 330 covers a region of the window member 320 without covering an upper region of the through-hole of the second reflective member 310. Accordingly, the fourth sealing member 330 does not impede propagation of light transmitted through the through-hole of the second reflective member and directed toward an upper region of the window member 320.

In addition, the fourth sealing member 330 may be formed along an edge of the window member 320 to cover a minimum area of the window member 320. Accordingly, the fourth sealing member 330 can maximize an exposed area of the window member 320. Accordingly, the fourth sealing member 330 can secure the window member 320 to the second reflective member 310 while maximizing a light exit area of the window member 320 to minimize reduction in light transmission through the window member 320.

The fluid treatment device 10 according to this embodiment may further include an adhesive material interposed between the fourth sealing member 330 and the window member 320 to firmly secure the fourth sealing member 330, the window member 320, and the second reflective member 310 to each other.

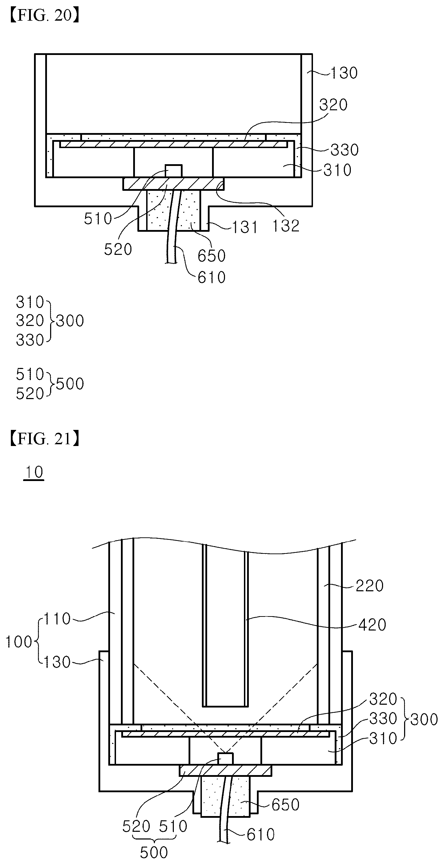

FIG. 19 is a plan view of the second housing 130 of the fluid treatment device 10 according to the embodiment of the present invention, with the light source unit 500 and the second reflector 300 disposed therein. FIG. 20 is a cross-sectional view of the second housing 130 of the fluid treatment device 10 according to the embodiment, with the light source unit 500 and the second reflector 300 disposed therein.

Referring to FIG. 19 and FIG. 20, when the light source unit 500 is disposed on the third seating unit 132, the lower surface of the substrate 520 may closely contact the inner lower surface of the second housing 130 placed inside the third seating unit 132.

A third sealing member 650 may be formed under the light source unit 500. The third sealing member 650 may cover a region of the electric wire 610 connected to the substrate 520 of the light source unit 500. In addition, the third sealing member 650 may fill the through-hole of the second protrusion 131 of the second housing 130. In this way, the third sealing member 650 may shield the inner space of the second housing 130 from an exterior space.

According to this embodiment, the third sealing member 650 may be formed of an elastic material. When the third sealing member 650 is formed of an elastic material, the third sealing member 650 can more effectively seal the through-hole of the second protrusion 131 of the second housing 130. For example, the third sealing member 650 may be formed of silicone.

Referring to FIG. 19 and FIG. 20, when the second reflector 300 is inserted into the second housing 130, an outer side surface of the fourth sealing member 330 may closely contact the inner side surface of the second housing 130.

Since the inner side surface of the second housing 130 closely contacts the outer side surface of the second reflector 300 and the window member 320 covers the through-hole of the second reflector 300, the light source unit 500 can be isolated from the inner space of the housing 100 in which a fluid flows.

Referring to FIG. 3, the inner space of the inner tube 420 defines a first flow path 141, which is a passage through which a fluid introduced through the inlet port 121 passes. In addition, the space between the housing 100 and the inner tube 420 defines a second flow path 142, which is a passage along which a fluid from the inner tube 420 is moved toward the outlet port 125.

In the fluid treatment device 10 according to this embodiment, the inner space of the housing 100 is divided into the first flow path 141 and the second flow path 142 by the inner tube 420 disposed inside the housing 100. Accordingly, the length of a flow path inside the housing 100 along which a fluid is moved from the inlet port 121 to the outlet port 125 is increased. As a result, the residence time of the fluid inside the housing 100 increases, thereby increasing the exposure time of the fluid to light from the light source unit 500. Accordingly, the fluid treatment device 10 according to this embodiment can more efficiently sterilize the fluid by increasing the exposure time of the fluid to sterilization light inside the housing 100.

FIG. 21 is an exemplary view illustrating the length of the inner tube 420 of the fluid treatment device 10 according to the embodiment.

The light source 510 of the light source unit 500 has a predetermined beam angle, which is the angle at which light is emitted from the light source, and an area within the beam angle may be designated as an illumination area. Within the illumination area of the light source unit 500, a fluid is exposed to light directly from the light source 510. Further, the irradiation area may include a concentrated sterilization zone. The concentrated sterilization zone is an area adjacent to the light source unit 500, has a higher light intensity than other areas, and has a predetermined sterilization power against contaminants. For example, the concentrated sterilization zone may be an area in which light emitted from the light source unit 500 directly irradiates a fluid, and may have a sterilization power of 90% or more against contaminants.

Referring to FIG. 21, a lower end of the inner tube 420 may be located within the irradiation area of the light source unit 500. Further, the lower end of the inner tube 420 may be located within the concentrated sterilization zone. That is, in the fluid treatment device 10 according to this embodiment, the inner tube 420 has a length that allows the lower end of the inner tube 420 to be located within the irradiation area or the concentrated sterilization zone. The lower end of the inner tube 420 is an end of the inner tube 420 facing the light source unit 500, among two longitudinal ends of the inner tube 420. The lower end of the inner tube 420 is formed with an opening through which a fluid is discharged from the first flow path 141 to the second flow path 142.

Since the lower end of the inner tube 420 is located within the irradiation area of the light source unit 500, all the fluid discharged from the first flow path 141 to the second flow path 142 passes through the irradiation area. Accordingly, when the fluid is discharged from the lower end of the inner tube 420, the fluid can be sterilized by direct exposure to light emitted from the light source unit 500.

Furthermore, when the lower end of the inner tube 420 is located within the concentrated sterilization zone, all the fluid discharged from the first flow path 141 to the second flow path 142 passes through the concentrated sterilization zone. Accordingly, all the fluid introduced into the fluid treatment device 10 can be sterilized by exposure to high-intensity light at the lower end of the inner tube 420.

According to this embodiment, since the light source unit 500 has a predetermined beam angle, the range of irradiation gradually increases with increasing distance from the light source unit 500. Accordingly, not only the lower end of the inner tube 420 but also the entire inner tube 420 may be located within the irradiation area or the concentrated sterilization zone. That is, the first flow path 141, which is an interior of the inner tube 42, may be irradiated with light directly from the light source unit 500.

Accordingly, a fluid may be primarily sterilized in the first flow path 141 by light directly from the light source unit 500. Here, the fluid may be sterilized by exposure to high-intensity light at the lower end of the inner tube 420 before being discharged to the second flow path 142.

In addition, the fluid discharged from the lower end of the inner tube 420 may be secondarily sterilized by exposure to light reflected from the inner wall of the first reflective member 220 while passing through the second flow path 142.

As such, the fluid treatment device 10 according to this embodiment can enhance sterilization efficiency by ensuring that a fluid introduced through the inlet port 121 is sterilized at least twice before being discharged to the outlet port 125.

Although it has been described that the first flow path 141 is irradiated with light directly from the light source unit 500 and the second flow path 142 is irradiated with light reflected from the first reflective member 220 in this embodiment, the present invention is not limited thereto. Since the inner tube 420 is formed of a light-transmissive material, the first flow path 141 may also be irradiated with light reflected from the first reflective member 220 and having passed through the inner tube 420. In addition, the second flow path 142 may also be irradiated with light emitted from the light source unit 500 to the first flow path 141 and having passed through the inner tube 420. That is, the first flow path 141 and the second flow path 142 may be irradiated with both light directly from the light source unit 500 and light reflected from the first reflective member 220.

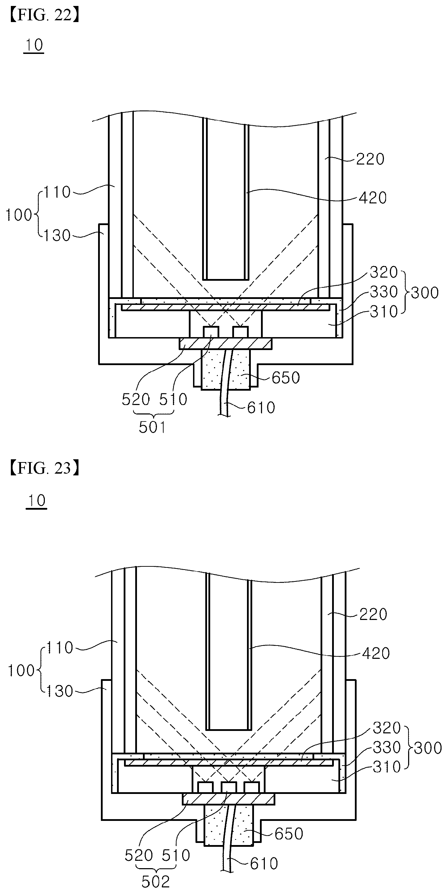

FIG. 22 is an exemplary view illustrating the length of the inner tube 420 when the fluid treatment device 10 according to the embodiment of the present invention includes a plurality of light sources 510. FIG. 23 is another exemplary view illustrating the length of the inner tube 420 when the fluid treatment device 10 according to present invention includes a plurality of light sources 510.

Referring to FIG. 22 and 23, a light source unit 501; 502 of the fluid treatment device 10 according to these embodiments may include a plurality of light sources 510. For example, the light source unit 501 may include two light sources 510, as shown in FIG. 22, and the light source unit 503 may include three light sources 510, as shown in FIG. 23.

Each light source 510 may have an irradiation area. Since the plurality of light sources 510 are placed at different locations on the substrate 520, the locations of respective irradiation areas are different from each other.

According to these embodiments, the lower end of the inner tube 420 may be located in a region where the plurality of irradiation areas formed by the respective light sources 510 overlap.

Referring to FIG. 22, the light source unit 500 may have two irradiation areas formed by the respective two light sources 510. Here, the lower end of the inner tube 420 may be located in a region where the two irradiation areas overlap.

Referring to FIG. 23, the light source unit 500 may have three irradiation areas formed by the respective three light sources 510. Here, the lower end of the inner tube 420 may be located in a region where all three irradiation areas overlap.

Although the light source unit 501; 502 is shown as including two or three light sources 510 in FIG. 22 and FIG. 23, the present invention is not limited thereto and the light source unit may include a greater number of light sources 510.

When the light source unit 500 includes a plurality of light sources 510, the lower end of the inner tube 420, through which a fluid is discharged, may be located in a region where two or more irradiation areas overlap. A region where the plurality of irradiation areas overlap is a region simultaneously irradiated with light from the plurality of light sources 510. Accordingly, the region where the plurality of irradiation areas overlap is irradiated with higher intensity light than other regions.

When the fluid treatment device 10 according to the present the embodiment includes a plurality of light sources 510, the lower end of the inner tube 420, through which a fluid is discharged, may be located in a region where two or more irradiation areas overlap. Accordingly, all the fluid discharged to the second flow path 142 after passing through the first flow path 141 of the inner tube 420 may be exposed simultaneously to light from the two or more light sources 510. Accordingly, the fluid treatment device 10 according to this embodiment can improve sterilization efficiency by ensuring that a fluid is exposed to high intensity light as the fluid flows from the first flow path 141 of the inner tube 420 to the second flow path 142.

Additionally, when the fluid treatment device 10 according to this embodiment includes a plurality of light sources 510, the lower end of the inner tube 420 may be located in a region where the largest number of irradiation areas overlap. Alternatively, when the fluid treatment device 10 according to this embodiment includes a plurality of light sources 510, the lower end of the inner tube 420 may be located in a region where all the irradiation areas overlap. Accordingly, the fluid treatment device 10 can improve sterilization efficiency by ensuring that a fluid passes through a region where the highest light intensity is concentrated.

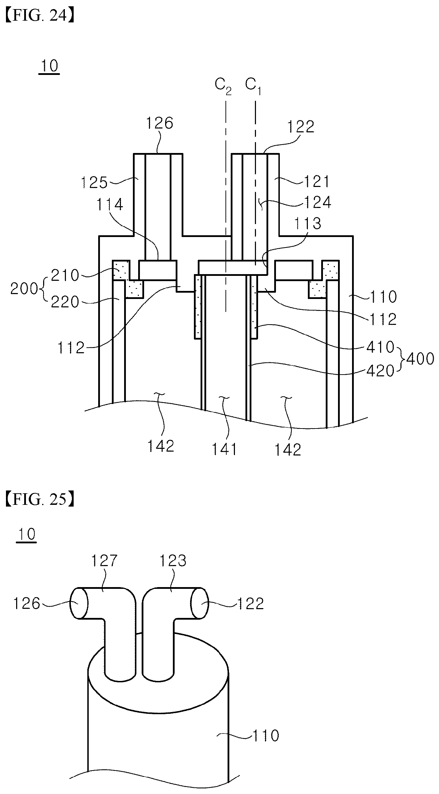

FIG. 24 is a view illustrating the location of the inlet port 121 of the fluid treatment device 10 according to the embodiment.

Referring to FIG. 3 and FIG. 24, the fluid treatment device 10 according to this embodiment may have a structure in which a central axis C1 of the inlet port 121 is offset from a central axis C2 of the inner tube 420.

Here, the central axis C1 of the inlet port 121 a straight line that passes through a center of the inlet passage 124 and is perpendicular to the upper surface of the first housing 110. The central axis C2 of the inner tube 420 is a straight line that passes through a center of the inner space of the inner tube 420 and is parallel to a longitudinal direction of the inner tube 420. In the fluid treatment device 10 according to this embodiment, at least a region of the inlet passage 124 may be located directly above the first flow path 141 of the inner tube 420.

Since the inflow hole 113 of the first housing 110 is formed on the side surface of the second seating unit 112, a fluid undergoes a change in direction as the fluid travels from the inlet passage 124 to the inflow hole 113 and from the inflow hole 113 to the first flow path 141 of the inner tube 420.

As such, since the central axis C1 of the inlet port 121 is offset from the central axis C2 of the inner tube 420, the fluid treatment device 10 according to this embodiment can prevent a fluid from traveling the shortest distance from the inlet port 121 to the first flow path 141 of the inner tube 420. That is, the travel distance of the fluid from the inlet port 122 to the inner tube 420 increases. In addition, the fluid undergoes a change in direction at least once, rather than traveling in a direction from the inlet port 122 to the inner tube 420.

Accordingly, the structure of the inlet port 121 and the inner tube 420 according to this embodiment enables reduction in fluid velocity by increasing the travel distance of the fluid from the inlet port 122 to the first flow path 141 of the inner tube 420 and ensuring that the fluid undergoes a change in direction as the fluid travels from the inlet port 122 to the first flow path 141 of the inner tube 420.

In addition, according to this embodiment, the diameter of the inflow hole 113 formed on the second seating unit 112 may be smaller than the inner diameter of the inlet port 121. Accordingly, the fluid pressure in the inflow hole 113 increases, resulting in decrease in fluid velocity in the first flow path 141 of the inner tube 420 compared to the fluid velocity in the inlet port 121. Accordingly, the time taken until both the first passage 141 and the second passage 142 are fully filled with the fluid, causing the fluid to be discharged to the outside of the fluid treatment device 10, increases.

Furthermore, since the inner diameter of the outlet port 125 is smaller than the diameter of the second flow path 142, the fluid pressure in the discharge hole 114 increases. As a result, the fluid velocity in the outlet port 125 decreases compared to that in the second flow path 142.

Accordingly, the fluid treatment device 10 according to this embodiment can improve sterilization efficiency by increasing the residence time of a fluid inside the first flow path 141 and the second flow path 142 and thus increasing the exposure time of the fluid to light from the light source unit 500.

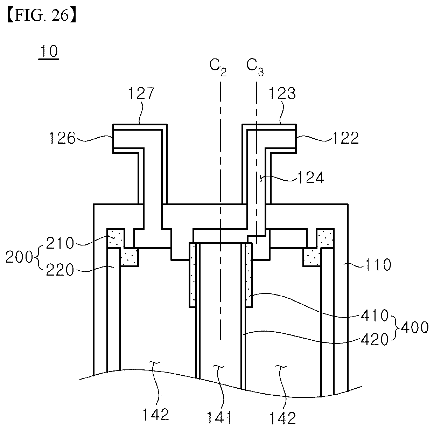

FIG. 25 and FIG. 26 are exemplary views illustrating other embodiments of the inlet port and the outlet port of the fluid treatment device 10 according to the embodiment of the present invention.

FIG. 25 is a view illustrating an external appearance of another embodiment of the inlet port and the outlet port of the fluid treatment device 10 according to the embodiment of the present invention. FIG. 26 is a cross-sectional view of a further embodiment of the inlet port and the outlet port of the fluid treatment device 10 according to the embodiment of the present invention.

Referring to FIG. 25 and FIG. 26, the fluid treatment device 10 may include a bent inlet port 123 and a bent outlet port 127. Accordingly, the fluid treatment device 10 may further include an additional section allowing a fluid introduced into the inlet port 122 to undergo a change in direction before reaching the first flow path 141 of the inner tube 420. Additionally, the fluid treatment device 10 may include an additional section allowing a fluid having passed through the second flow path 142 to undergo a change in direction before being discharged through the outlet port 126. That is, the fluid treatment device 10 may include an increased number of sections allowing a fluid introduced into the inlet port 122 to undergo a change in direction before being discharged through the outlet port 126. The velocity of a fluid decreases when the fluid undergoes a change in direction. Accordingly, the bent inlet port 123 and the bent outlet port 127 can increase the residence time of the fluid inside the fluid treatment device 10, thereby improving fluid treatment efficiency.

Referring to FIG. 26, the fluid treatment device 10 may have a structure in which a central axis C3 of an inlet port 123 is offset from a central axis C2 of the inner tube 420, as in FIG. 24. However, this embodiment differs from the embodiment illustrated in FIG. 24 in that an inlet passage 124 is not placed directly above the first flow path 141 of the inner tube 420. In FIG. 26, the central axis C3 of the inlet port 123 corresponds to the central axis of the inlet passage 124 of the inlet port 123, which is parallel to the inner tube 420. That is, the central axis C3 of the inlet port 123 may be parallel to the central axis C2 of the inner tube 420. Since the central axes C2, C3 are offset from each other, the distance between the inlet port 121 and the inner tube 420 increases, resulting in increase in travel distance of a fluid.

In the embodiments of the present invention, it has been described that the fluid treated by the fluid treatment device is water. However, the fluid treated by the fluid treatment device is not limited to water. The fluid treated by the fluid treatment device according to the present invention may include not only water but also various liquids or gases that need to be sterilized.

Although some embodiments have been described herein with reference to the accompanying drawings, it should be understood that these embodiments are provided for illustration only and are not to be construed in any way as limiting the present invention. Therefore, the scope of the present invention should be defined by the appended claims and equivalents thereto.

## Claims

1. A fluid treatment device comprising:
a housing comprising an inlet port through which a fluid is introduced and an outlet port through which the fluid is discharged;
an inner tube unit disposed inside the housing, having a smaller inner diameter than the housing, and open at a lower end thereof; and
a light source unit emitting light to an inner space of the housing,
wherein an inner space of the inner tube unit defines a first flow path,
wherein a space between the inner tube unit and the housing defines a second flow path,
wherein the fluid introduced through the inlet port flows to the outlet port after passing through the first flow path and the second flow path,
wherein the light source unit is disposed to face the lower end of the inner tube unit,
wherein the lower end of the inner tube unit is located within an irradiation area in which the fluid is exposed to light directly from the light source unit, and
wherein a central axis of the inlet port lies on a different line than a central axis of the inner tube unit.

2. The fluid treatment device according to claim 1, further comprising:
a first reflector closely contacting an inner wall of the housing and spaced apart from the inner tube unit,
wherein the first reflector comprises a material reflecting light from the light source unit.

3. The fluid treatment device according to claim 2, wherein
the first reflector is formed by injection molding and is inserted into the housing.

4. The fluid treatment device according to claim 1, wherein
the inner tube unit comprises an inner tube defining the first flow path and formed of a light transmissive material.

5. The fluid treatment device according to claim 1, wherein
the housing comprises a stepped seating unit on which the inner tube unit is seated through insertion of an upper end of the inner tube unit, and
the seating unit is formed with an inflow hole connected to the inlet port.

6. The fluid treatment device according to claim 5, wherein
the seating unit comprises a first step and a second step located outside the first step,
the first step has a lower height than the second step, and
the upper end of the inner tube unit is inserted into the second step of the seating unit to be seated on the first step.

7. The fluid treatment device according to claim 6, wherein
the inflow hole is formed on the first step of the seating unit.

8. The fluid treatment device according to claim 7, wherein
the inflow hole is formed on the first step of the seating unit to extend to an inner wall of the housing located inside the seating unit.

9. The fluid treatment device according to claim 6, wherein
a diameter of the inflow hole is smaller than an inner diameter of the inlet port.

10. The fluid treatment device according to claim 1, further comprising:
a second reflector disposed between the inner tube unit and the light source unit inside the housing,
wherein a region of the light source unit is placed inside the second reflector.

11. The fluid treatment device according to claim 10, wherein
the second reflector comprises:
a reflective member formed with a through-hole and reflecting light from the light source unit; and
a window member disposed on an upper surface of the reflective member to cover the through-hole of the reflective member, the window member being formed of a material transmitting light from the light source unit, and
a region of the light source unit is disposed in the through-hole of the reflective member of the second reflector.

12. The fluid treatment device according to claim 11, wherein
the second reflector comprises a sealing member covering at least a region of a side surface of the reflective member of the second reflector, a region of an upper surface of the reflective member, and an upper edge of the window member.

13. The fluid treatment device according to claim 1, wherein
each of the inlet port and the outlet port has a bent shape.

14. The fluid treatment device according to claim 1, wherein
the irradiation area comprises a concentrated sterilization zone having a sterilizing power of 90% or more against contaminants contained in the fluid.

15. The fluid treatment device according to claim 1, wherein
the light source unit comprises a plurality of light sources.

16. The fluid treatment device according to claim 15, wherein the lower end of the inner tube unit is located in a region where irradiation areas of at least two light sources overlap.

17. The fluid treatment device according to claim 9, wherein
the housing comprises:
a first housing in which the inner tube unit is disposed; and
a second housing in which the second reflector and the light source unit are disposed,
the first housing and the second housing having different outer diameters, and
the first housing is coupled to the second housing through insertion of a region of the first housing into the second housing.

18. The fluid treatment device according to claim 1, further comprising:
a drive unit disposed outside the housing and comprising a drive circuit controlling operation of the light source unit.
